# EUROPEAN PATENT SPECIFICATION

(11) **EP 3 960 039 B1**
(45) Date of publication and mention of the grant of the patent: **25.10.2023**
(21) Application number: 21192795.9
(22) Date of filing: 24.08.2021
(51) Int. Cl.: A47G 9/02, A61H 39/04, A61M 21/02

(54) **WEIGHTED DUVET WITH DIFFERENT WEIGHT ZONES**
GEWICHTETE BETTDECKE MIT VERSCHIEDENEN GEWICHTSZONEN
COUETTE LESTÉE AVEC DIFFÉRENTES ZONES DE POIDS

(30) Priority: 24.08.2020 DK PA202070549
(43) Date of publication of application: 02.03.2022
(73) Proprietor: Zibo Care Denmark ApS, 8700 Horsens (DK)
(72) Inventor: DENKER, Jeannette Buchard, 6200 Aabenraa (DK)
(74) Representative: Rasmussen, Martin Hoffgaard

(56) References cited:
- EP-B1- 2 257 260
- WO-A1-2014/116163
- WO-A1-2014/166557
- WO-A1-2017/194456
- WO-A1-2019/177523
- JP-A- 2001 112 585
- US-B2- 9 089 228

## Description

### FIELD OF THE INVENTION

The present invention relates to the field of aids for stimulating a duvet user. More specifically, the invention relates to an aid comprising weight fillings for stimulating the senses of a duvet user.

### BACKGROUND OF THE INVENTION

People having trouble finding rest, relaxing or sleeping may benefit from external stimuli offering a sensory input to focus on. One such external stimuli can be provided by a weighted duvet or blanket which can be wrapped around a person to provide pressure. Such a blanket may be especially helpful for people suffering from depression, ADHD, dementia, autism, sensorimotor disorders, stress, or other conditions that may provoke the unwanted effect of being unable to relax or sleep.

A weighted duvet is, as the name suggests, a duvet or blanket weighing more than the weight of a traditional duvet through the addition of weights or by being made from heavier materials. For the best effect of a weighted duvet, it is important that it is comfortable for the duvet user, that the weight and weight distribution accommodates the size of the duvet user, e.g. an adult or a child, and that the duvet adjusts to the shape of the body of the duvet user.

In addition to providing a sensory input for the duvet user to focus on and helping the duvet user identify the boundaries of the body, the deep pressure to the proprioceptive body sense provided by a weighted duvet and the stimulation of the tactile sense trigger the release of hormones, including oxytocin. Oxytocin has important physical and psychosocial effects including making a person feel more calm and safe.

Various types of weighted blankets exist with different objects added to create the weight. In one known variant, pockets filled with small, loose weights such as balls are added to a fabric. An alternative variant of weighted blankets is to sew small rings into pockets or directly into the fabric of the duvet to avoid them moving about. Other variants use chains to provide weight instead of loose balls to avoid the problem of bunching while maintaining flexibility. In such variants, the chains will be attached in both ends of the duvet and may either be allowed to move freely between these points of attachment or they may be placed within channels in the duvet, to restrict their movement to some extent while still allowing them to follow the contours of the body of the duvet user.

However, subjecting the body to weight for an extended period of time can also pose problems as it puts pressure and strain on the joints which might force them out of the naturally relaxed position and could for example cause lead to undesirable flexion of joints of the users such as the ankles of the users. Forced flexing of the joints might be uncomfortable for the user and, over prolonged periods of time, may cause pain and even injury caused by strain and wear to the joints, e.g. arthritis. Furthermore, applying weights to the user also makes it harder for the user to move putting additional strain on the muscles of the user. In some instances, this is beneficial as it helps keep the user still and calm, however some muscles must remain active even when the user is relaxing such as the muscles used for moving the lungs of the user and allowing them to breathe. Weighing down the body of the user in a manner that makes breathing more cumbersome is particularly problematic when the weighted duvet is used to treat a condition which in itself is associated with troubled breathing, e.g. anxiety.

One example of prior art is disclosed in WO 2017/194456 A1 which discloses a weight blanket which has a number of chains. The chains run from the head end to the foot end, and the ends of the chains are connected to the head end and the foot end by the use of bands. The document discloses a weighted duvet according to the preamble of claim 1.

Hence, there is a need for a weighted duvet that can provide the user with the calming effect without adverse effects caused by applying weights to the body.

### SUMMARY OF THE INVENTION

It is the aim of the present invention to provide a weighted duvet capable of applying pressure to the body of a user while applying a minimum of pressure to most vulnerable or sensitive regions of the body of the user.

This is achieved by a weighted duvet comprising:
- a first edge positioned opposite a second edge and a third edge positioned opposite a fourth edge;
- a first fabric layer, a second fabric layer;
- at least one duvet section in the central weighted zone of the weighted duvet;
- at least one weight element placed between said first and said second fabric layer and within said at least one duvet section;
the weighted duvet further comprises a first lightweight zone adjacent to and spanning the length of said first edge and a second light weight zone adjacent to and spanning the length of said second edge; and wherein the first lightweight zone extends at least 10 cm from the first edge and the second lightweight zone extends at least 10 cm from the second edge.

By a weighted duvet is understood a flexible material with weighted objects between sheets of fabric as such it could be any type of blanket, quilt or other cover.

The weighted duvet has at least four sides, preferably being a rectangle when stretched out on a flat surface. The first and second side oppose each other, and the first lightweight zone being adjacent to the first edge is thus also opposite of the second lightweight zone being adjacent to the second edge.

The weighted duvet consists of at least two pieces of fabric creating the top and the bottom surfaces of the weighted duvet and between which the at least one duvet section is located. The at least one duvet section is delimited by stitches holding the first and second fabric layers together.

The duvet section for containing at least one weight element is located in the central weighted zone which is the full middle region between the first and second lightweight zones. The duvet section may be all of this central weighted zone. However, in a preferred variant of the invention there will be multiple duvet sections created by multiple stitches that help contain multiple weight elements. The weight elements may take different forms, in one version they may be balls that can move freely within the respective duvet sections, in other versions the weight elements may be connected such that they affect the movement of each other, while they are still restricted to their respective duvet sections. In some variants of the invention, multiple types of weight elements may be used in the same weighted duvet - multiple types of weight elements may be in the same duvet section or they may be divided in different duvet sections. Not all duvet sections need to contain a weight element but at least one of them does, other duvet sections may be unweighted. Similarly, different duvet sections may have the same type of weight elements but with different average mass to allow for varying pressure exerted by different regions of the weighted duvet.

The duvet sections may have different geometries, e.g. they may be channels spanning the full length between the first and second lightweight zones or they may be smaller pockets. Different geometries are beneficial for accommodating different types of weight elements.

By a lightweight zone is understood a zone which has a lower average mass than the average mass of the central weighted zone. In other words, the lightweight zones will on average exert a lower force per area due to gravity than will on average be exerted per area in the central weighted zone. This further means that the average mass per area of the lightweight zone is lower than the average mass per area of the total weighted duvet. The lightweight zones will naturally comprise some amount of mass from the fabric of the weighted duvet. It may further be supplied with weight elements which can be lighter and/or sparser than in the central weighted zone.

The lightweight zone are regions with little mass and therefore exert only little force onto the duvet user under the lightweight zones. Minimising the force exerted in the lightweight zones ensures that stress will not be applied to the parts of the body under the lightweight zones. The lightweight zones are located at opposite ends of the weighted duvet and is intended to be placed over the feet and chest of the duvet user respectively. If a heavy region of a weighted duvet is placed over the feet of the duvet user, it might lead to unintended flexion of the joints of the ankles and toes which may cause discomfort and pain, and even long-term issues for the user if the weighted duvet is used over longer periods of time. This is in particular an issue for users that sleep with the weighted blanket and thus risk putting extended strain on the joints of their feet.

The lightweight zone across the chest of the user lowers the amount of muscle-work that the user needs to do to breathe. Muscles are required to compress and expand the lungs to provide air into the lungs during the process of breathing, this is done by the diaphragm assisted by chest and stomach muscles. While some weighted parts of the weighted duvet may still be draped across the body of the duvet user in regions of the body where muscles contributing to the breathing movements are present, alleviating the pressure caused by weights at the top of the chest will still help easing the process of breathing. Alleviating the pressure at the top of the chest is done by the lightweight zone at the end of the weighted duvet. This is especially important when the weight duvet is used to calm a duvet user who suffers from psychological disorders that include episodes where they already have trouble breathing. While the weighted duvet has a calming effect, it is problematic if it makes it difficult for users to breathe, in some cases it may lessen the calming effect and in the worst cases it may be dangerous to the duvet user. Furthermore, the lightweight zone, in the region of the chest of the duvet user, will also benefit the duvet users with no breathing difficulties as it allows more versatile use of the weighted duvet improving the comfort for the duvet user. By having a lightweight zone near the chest and neck area of the duvet user, the user can pull the weighted duvet close to the neck and chin without experiencing weight on the throat. At the same time the duvet user can obtain a snug fit of the weighted duvet around the neck and shoulders providing comfort in the feeling of being embraced by the weighted duvet; this can also help ensure that the duvet user can stay warm on the neck and shoulder area.

The number of sections in the duvet may vary allowing for more fine-tuning of the weight distribution. Each section may comprise one or more discrete segments of connected weights and these may vary in make. Thereby, it is possible to make weighted duvets customised to the needs of the duvet user, for example by making the weighted duvet heavier in a band across the middle, at the legs or on the sides whereby it is more difficult for the blanket to slide off a sleeping duvet user.

The total weight of the weighted duvet is not fixed as different users may benefit from different amounts of weight, e.g. a child might require a less heavy weighted duvet to avoid stress to their body while an adult might require a heavier weighted duvet to obtain the benefits from the weight stimuli. Different variants of the weighted blankets may have total weights of about 4 kg, 6 kg, 8 kg, 10 kg, 12 kg, and 14 kg.

As previously mentioned, the lightweight zones ensure that a minimum of stress is being applied to the duvet user in the regions of the lightweight zones, e.g. at the feet and in the chest area. Ideally, the extend of the first and second light weight zones are such that they are gentle on the relevant regions, e.g. ankles and breathing muscles, while providing a large area with weight stimulus so that the user gains the benefits of the weighted duvet in other regions of the body. Hence, different costumers with differing anatomy and using the weighted duvet for treatment of different conditions may benefit from different extends of the lightweight zone.

In some variants of the invention, the lightweight zones extend at least 15 cm from the first and second edge respectively. In another variant of the invention, the lightweight zones extend at least 20 cm from the first and second edge respectively. In yet another variant, the lightweight zones extend at least 30 cm from the first and second edge respectively.

It is to be understood that the first lightweight zone and the second light weight zone do not need to have the same extend. For example, the first lightweight zone may be shorter than the second lightweight zone allowing the users to orient the weighted duvet differently depending on their needs in the position they prefer to be in when using the weighted duvet.

In an embodiment of the invention, the first and second lightweight zone are placed at the ends of the weighted duvet with respect to its length and they span the width of the lightweight duvet.

Most duvet users will use the weighted duvet in such a way that the length, i.e. the longest side of the weighted duvet, is parallel to the body of the user when they rest under the weighted duvet, while the width is draped across their body. When the weighted duvet is used in this intended manner, having the lightweight zones located at the ends of the weighted duvet with respect to its length, it ensures that the lightweight zones will be placed across the feet in one end and the chest and shoulders of the duvet user at the other end. These are the body parts of the duvet user which will benefit the most from having lessened strain from the weight of the weighted duvet.

In an embodiment of the invention, neither the first lightweight zone nor the second light weight zone contains any weighted element.

By having no weighted element in the lightweight zones, the force exerted by these lightweight zones onto the user is minimised as much as possible. The lightweight zones will still have mass from the fabric and potential filling which helps keeping the user warm and comfortable, but compared to the weighted elements the lightweight zones will be substantially lighter and will not encumber the breathing of the user or stress the joints of the user when they rest under the lightweight zones of the weighted duvet. Comprising no weighted elements in the lightweight zones is a way in which to minimise the exerted force as much as possible.

In an embodiment of the invention, the duvet sections are delimited by sectioning stitches extending through the first fabric layer and the second fabric layer and the first lightweight zone and the second light weight zone are delimited by zone edges.

The duvet sections are made from stitching together neighbouring layers of the weighted duvet whereby mutual borders are made between adjacent duvet sections. In an embodiment where there is a first and a second fabric layer, the stitches will extend through both of the fabric layers. If a third fabric layer is added onto the weighted duvet, the sectioning stitches will in a preferred embodiment extend through all three layers of the fabric. However, in other embodiments of the invention, the sectioning stitches will extend through only the fabric layers adjacent to the weight elements. If further layers beyond three are added to the weighted duvet, it is similarly preferable to have the sectioning stitches extend through all layers. However, variants within the scope of the invention may have the sectioning stitches extend through some, but not all, of the fabric layers.

Having the duvet sections extending through all of the fabric layers, it has the benefit of holding together all layers and keeping their positions relative to each other the same such that they will not slide relative to each other. If they were allowed to slide, this might cause lumping of the fabric of some of the layers which may be annoying to the duvet user.

Both the sectioning stitches and the zone edges further provide the benefit of restricting the movement of weighted elements as they cannot move past the sectioning stitches or zone edges. Thus, in a preferred variant of the invention, the sectioning stitches and zone edges are so closely spaced that weight elements cannot exit a duvet section between stitches.

In an embodiment of the invention, weight elements having at least two different average weights are placed in at least two different duvet sections.

By having multiple different types of weight elements confined to different duvet sections, it is possible to adjust and customize the weight as well as the tactile stimuli to the needs of the duvet user.

Some users may benefit from having some regions heavier than others, e.g. by having heavier weight elements placed in the duvet sections in some regions of the weighted duvet. Additional stimuli may be obtained by having intermittent rows of heavier and lighter connected weights in neighbouring segments. Having heavier connected weight elements near the edge of the weighted blanket may provide a different benefit of making it more difficult for the weighted duvet to slide off the duvet user if they move in their sleep. Alternatively, having the weighted duvet heavier centrally and lighter at the edges, it allows the weighted duvet to provide weight where it is most needed while once more protecting the joints of extremities of the duvet user, e.g. if an arm is placed to the side of the user. Less weight at the edges of the weighted duvet also makes it easier for the duvet user

Similarly, having different types of weight elements, e.g. chains and beads on a string and/or loose beads and/or rings sown directly onto one of the fabric layers, in different duvet sections, it will result in both different weight and different tactile inputs.

In different variants of the invention, different weights of the weight elements are used. For connected weight elements, the following approximate variants of weights per weight element per duvet section are used: 0.2 kg, 0.4 kg, and 0.6 kg. Other mass per duvet section may also be used, the numbers are given to indicate the likely ranges and variations.

In an embodiment of the invention, there are at least two duvet sections, and one of them comprising at least one weight element, thereby being a weighted section, and another containing no weighted element.

Having duvet sections with no weight elements along with weighted sections has similar benefits to having duvet sections with different weights of weighted elements, e.g. allowing varying tactile stimuli, customising the regions receiving most stimuli and engineering how the weighted blanket might move during use. In addition, having duvet sections with no weight elements makes it possible to provide these benefits without needing to increase the total weight of the weighted duvet, thereby obtaining said benefits for users who need a lighter weighted blanket.

In an embodiment of the invention, at least one of said at least one weight elements is a connected weight element.

By connected weight elements is understood multiple elements having mass being connected in such a way that their position influences each other. This influence can be direct such as is the case with a chain link directly connected to neighbour chain links. Alternatively, the connection can be mediated through a connection mediator, e.g. a string on which beads can be drawn onto, a band which weights can be clasped onto, or a sheath which weights can be placed inside, in these cases the positions of the weight elements and the weight mediator relate to each other even though they have more freedom than the directly linked elements.

The mutual influencing of the connected weight elements provides a benefit as the placement of the weight within the weighted blanket thus becomes easy to guide and restrict. Connected weight elements may be attached in few points, e.g. end points at the middle or intermittently and the movement of the other weight element will be restricted too. This means that the weight elements will not be able to bunch up unintentionally in a small region of a duvet section when the weighted duvet is moved. This limits the risk of the weights falling off of the duvet user and not providing the intended effect as may happen with loose weight elements restricted only by the sectioning stitches.

In an embodiment of the invention, at least one of the connected weight elements is a chain.

Chains comprise mutually linked objects whereby their movement within the weighted duvet becomes coherent. Chains are readily available in various sizes and made from various materials, e.g. metal or plastic. This makes them a cost-effective choice.

Furthermore, chains are easily divided into discrete segments of various lengths by simply removing a link and this requires no further treatment of the exposed ends.

Additionally, the structures of chains, i.e. changes in loop direction and regions of overlapping links, means that chains inherently supply the tactile sense with the stimuli needed to trigger the oxytocin hormone.

In an embodiment of the invention, the weighted duvet comprises retaining means for guiding the weight distribution within said at least one duvet section.

By retaining means are understood features capable of partially restricting the movement of the discrete segments of connected weight elements. These may take various forms and may allow the discrete segments of the connected weight elements different degrees of freedom in their movement.

The retaining means may take many different forms, for example they may be separate means such as loops wound around connected weight elements, it may be stitches holding down the connection mediator between connected weighted elements, and it may be glue attaching weight elements to the first or second fabric layer of the weighted duvet. The sectioning stitches and/or the zones edges may also function as retaining means by blocking the movement of the weight elements or by the weight elements being sown onto the first and/or second fabric layer by the sectioning stitches and/or zones edges themselves.

In some variants of the invention, multiple different types of retaining means may be used simultaneously.

In an embodiment of the invention, the retaining means comprises at least one point fastener.

Point fasteners can take various forms such as being single points where the discrete segments are sown into the weighted duvet or it may be straps in which the weight elements can be threaded. Such straps may either be closed loops attached to the weighted blanket or they may include opening means allowing the straps a tighter fit around the connected weight elements as they do not need to be able to move within them. Point fasteners may also take other forms such as hooks or clasps.

The point fasteners have the benefit of ensuring weight in specific points, i.e. where the connected weight elements are fastened to the weighted duvet. Although it is important that the connected weight elements can move and adjust to the shape of the body of the duvet user, it may be preferable to ensure weights at specific points or at specific positions in relation to each other. Point fasteners give control over the weight distribution in specific points while allowing freedom of movement of the connected weight elements in others.

Point fasteners also ensure that it is known exactly where most wear will take place and makes it possible to reinforce these points to decrease the risk of tears and prolong the lifetime of the weighted duvet.

In an embodiment of the invention, the weighted duvet comprises releasable connection means allowing it to be divided into at least two smaller weighted duvets. In an embodiment of the invention, the releasable connection means is at least one zipper running across the entirety of said weighted duvet parallel to at least one of said duvet sections.

By a releasable connection means is to be understood a way to attach two parts of a weighted duvet and detach them from each other again. This may be done by a zipper, but other connection means would also be possible, such as buttons or hook-and-loop strips, or simply straps for tying two parts together.

Weighted duvets can be difficult to clean as they may become too heavy for washers or dryers to handle. Thus, being able to divide it into smaller segments, which can be washed separately, provides the weighted duvet with sanitary advantages. This is important both for private users and institutions. Especially at hospitals and mental care institutions where sanitary requirements are very high, easy washing is an important feature. Items that cannot be thoroughly cleaned are simply not feasible as it may compromise the safety of patients by increasing risks of spreading diseases.

Furthermore, being able to divide the weighted duvet into smaller segments gives it more versatility, for example if it is used intermittently by a single and multiple users or if a duvet user wishes to have a weighted blanket only on part of the body while having an un-weighted blanket on another part. Separate parts of the weighted duvet may be weighted similarly, but they may also comprise different weight elements allowing different regions to be customised and changed for different use situations while still having a large weighted duvet. This allows more specialised treatment while not needing to exchange the entire weighted duvet, and thereby keeping costs for the duvet user down.

In an embodiment of the invention, there is filling material present between the first fabric layer and the second fabric layer.

By filling is understood a material which is compactable under pressure and which is flexible, e.g. natural or synthetic fibres, wool, or pieces of foam. Such a layer of filling may provide other features such as insulation.

Providing filling between the first fabric layer and the second fabric layer makes it possible to make the weighted duvet warmer which is practical in colder climates.

Placing filling within the same layer as connected weight element may also provide a further, but mild, restriction to the movement of the discrete segments of the connected weight elements. In addition, the filling may also provide padding around the connected weight elements if it is desirable to have a more even distribution of the pressure. Filling placed in the same layer as the weight elements also allows a more level height of the weighted duvet as the amount of filling may be adjusted to the height of the connected weight elements themselves. These effects are adjustable in the production by choice of the type and the amount of filling provided between the fabric layers, whereby the weight duvet becomes more customisable. For example, it might be desirable for the connected weight elements to provide a textural difference in height as well, in which case less filler may be used.

Some duvet sections may be without filling material. This may for example provide the weighted duvet with channels which have no insulating filling whereby the skin of the user is allowed to breathe through the weighted duvet while it still provides good heating properties.

### SHORT LIST OF THE DRAWINGS

In the following, example embodiments are described according to the invention, where:
Figs. 1a-1b illustrate a person under a weighted duvet in accordance with the invention.
Fig. 2 is a weighted duvet according to the invention laid out flat and seen from the top.
Fig. 3 is a cross-sectional, semi-exploded view of a duvet section of a weighted duvet according to the invention illustrating the layers of the duvet.
Figs. 4a-4d show different possible types of connected weight elements that may be used in the weighted duvet.
Figs. 5a-5e illustrate various retaining means for keeping connected weight elements in place inside the duvet sections.
Figs. 6a-6f illustrate various versions of the weighted duvet where weights are distributed in the duvet sections in different manners.

### DETAILED DESCRIPTION OF DRAWINGS

In the following, the invention is described in detail through embodiments thereof that should not be thought of as limiting to the scope of the invention.

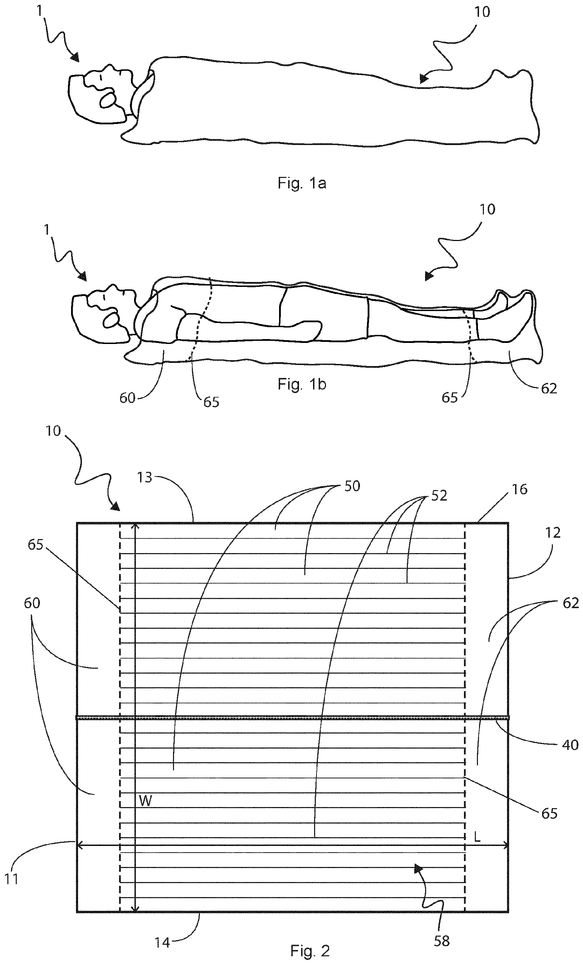

Fig. 1a shows the invention in use. A duvet user 1 rests under a weighted duvet 10 according to the invention. The weighted duvet 10 applies a pressure to the part of the duvet user 1, which is under weighted sections 55 of the weighted duvet 10; this deep pressure helps the duvet user 1 feel safe and relaxed.

Although Figs. 1a and 1b show a sleeping duvet user 1 lying under the weighted duvet 10, the weighted duvet 10 may also be used in other contexts such as wrapped around the shoulders of a duvet user 1 sitting upright or draped across the lap of a sitting duvet user 1.

As shown in Fig. 1a, the weighted duvet 10 may be covered in bedding to protect it and decrease the need for washing of the weighted duvet 10 itself. Hence, during use the weighted duvet 10 may resemble a common, unweighted duvet.

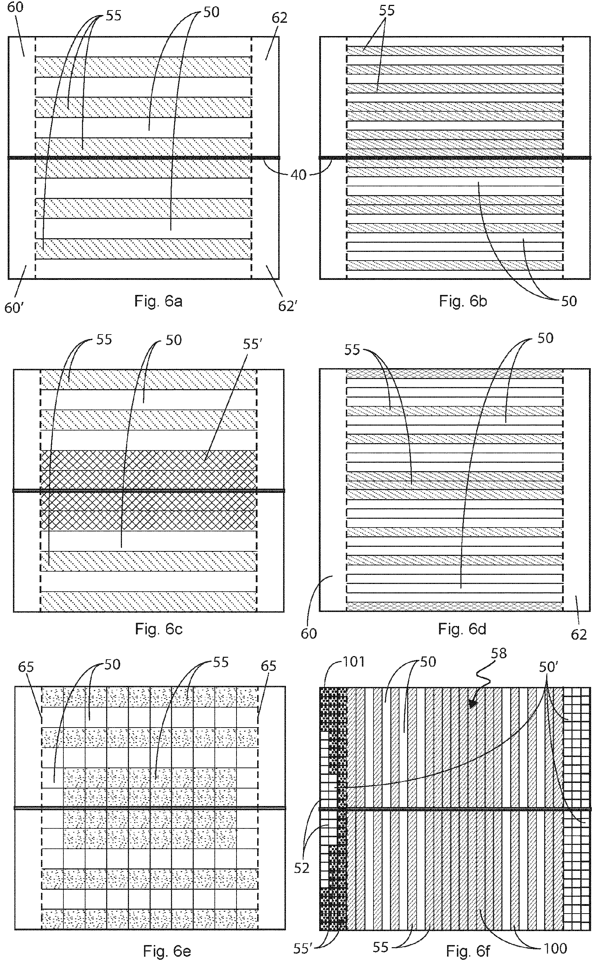

Fig. 1b shows the invention in use in a semi-transparent view, where the features of the duvet user 1 and different regions of the weighted duvet 10 are illustrated, note that the at least one weight element 100 and at least one duvet section 50 to contain the at least one weight element 100 is not illustrated in Fig. 1b - see instead Figs. 2 and 6a-6f for illustrations of these features. When the duvet user 1 is lying on his back, as illustrated in Figs. 1a and 1b, anything draped across them will apply a pressure to their feet forcing their ankles into a flexing position and straining the joint. Similarly, anything draped across the chest will increase the work that the muscles are required to do to allow the duvet user 1 to breathe. Fig. 1b shows the presence of a first lightweight zone 60 at the chest of the duvet user 1 and a second lightweight zone 62 at the feet of the duvet user 1. The first 60 and second lightweight zone 62 ensure that the duvet user 1 can be fully covered by a part of the weighted duvet 10 which will keep them warm and comfortable without adding excessive weight and thereby pressure to regions where it may cause discomfort, e.g. the neck, chest, and feet. This has the benefit of allowing the duvet user 1 to breathe freely and to not have their ankles stressed by the presence of weight elements while still having a full cover. The fabric and possibly filling of the cover will of course add some mass on their own, however, this is significantly less than in the weighted sections 55, thus applying much less force to the duvet user 1 under the first 60 and second light weight zone 62. In some embodiments of the invention, the first 60 and/or second lightweight zones 62 may also comprise lightweight elements 101 ensuring that their density and distribution maintain a lower average weight per area in the first 60 and second lightweight zone 62, respectively, than in the central weighted zone 58. These lightweight elements 101 may, same as the weight elements 100, take different forms such as loose lightweight elements 101 such as granules or small balls or they may be connected lightweight elements 101 such as chains or strings with beads on them.

Fig. 2 is an illustration of the weighted duvet 10 according to one embodiment of the invention shown without any bedding on. The weighted duvet 10 is shown stretched out on a flat surface. In a preferred embodiment of the invention, the weighted duvet 10 is rectangular as shown in Fig. 2. Thus, the weighted duvet 10 has a first edge 11, a second edge 12, a third edge 13, and a fourth edge 14. The first 11 and second edge 12 are located across from each other as is the third 13 and fourth edge 14. In a preferred embodiment of the invention, the first 11 and second edge 12 have the same length considered the width W of the weighted duvet 10. Similarly, the third 13 and fourth edge 14 have the same length, being considered the length L of the weighted duvet 10. In a preferred embodiment, the width W of the weighted duvet 10 is shorter than the length L of the weighted duvet, i.e. the first 11 and second edge 12 are shorter than the third 13 and fourth edge 14. In another embodiment of the invention, the first 11, second 12, third 13 and fourth 14 edges of the weighted duvet 10 may all have the same length.

In other embodiments of the invention, the weighted duvet 10 may have additional edges. In some embodiments of the invention, the weighted duvet 10 may be equipped with edging 16 in the form of one or more strips of cloth wrapped and sown around the edges of the of the weighted cover 10 holding together the various layers of fabric. Such edging 16 collects the edges of the various layers of the weighted duvet 10 keeping them together and protecting the edges of the layers themselves from fraying or other sorts of damage through wear.

In different embodiments, the weighted duvet 10 may have different dimensions. In a preferred embodiment, the weighted duvet 10 is around 140 cm x 200 cm, i.e. the width W being 140 cm and the length L being 200 cm as the first 11 and second edge 12 are 140 cm long and the third 13 and fourth edge 14 are 200 cm long. In other embodiments, one or both dimensions of the weighted duvet 10 may be different, for example 200 cm x 200 com, 100 cm x 140 cm, 140 cm x 220 cm, 200 x 220 cm, 220 cm x 220 cm, or 240 cm x 220 cm.

The weighted duvet 10 is divided into a number of duvet sections 50, these duvet sections 50 are defined by the sectioning stitches 52. The sectioning stitches 52 extend through all layers of the weighted duvet 10 and are thus visible on both sides of the weighted duvet 10. The weight elements 100 (not shown in Fig. 2) are confined within duvet sections 50. There may be weight elements 100 in each of the duvet sections 50, in which case they may be denoted weighted section 55, or they may be present in only some of them, thus leaving some of the duvet sections 50 unweighted. Similarly, there may be different types of weight elements 100 in different duvet sections 50.

The duvet sections 50 may be long channels as illustrated in Fig. 2, but they may also take any other shape or take multiple different shapes across the weighted duvet 10. For example, larger sections may be bordered by narrow sections, where the narrower sections have no connected weight elements 100 in them, thus providing channels with increased breathability. An alternative example is to have many smaller duvet sections 50 in regions where additional weight is preferable. Channels as illustrated in Fig. 2 are preferable when the weight elements 100 are connected weight elements 110, e.g. chains or beads held together by a string. In other embodiments of the invention, the weight elements 100 may be loose, e.g. beads or dried grains, in such embodiments smaller duvet sections 50 are preferable for restricting the movement of the weight elements 100 within the duvet sections 50, thereby ensuring that the weight elements 100 will not pool in one end of the weighted duvet 10.

The weighted duvet 10 has a first lightweight zone 60 adjacent to the first edge 11 and delimited towards the centre of the weighted duvet 10 by a zone edge 65 taking the form of a seam. The weighted duvet 10 has a second lightweight zone 62 adjacent to the second edge 12 and delimited towards the centre of the weighted duvet 10 by a zone edge 65 taking the form of a seam.

In other words, the first 60 and second lightweight zone 62 are located at the ends of the weighted duvet with respect to its length L and spanning the width W of the weighted duvet. Said differently, in a preferred embodiment of the invention, the first 60 and second lightweight zone 62 are perpendicular to the length L of the weighted duvet and located at its opposing ends.

In relation to the duvet user 1, the first 60 and second lightweight zone 62 are placed at the ends of the weighted duvet 10 such that the first 60 and second lightweight zone 62 are placed across the body of the duvet user 1. In other words, the length L of the weighted duvet 10 is parallel to the body of the duvet user 1 when the weighted duvet 10 is in use and the width W of the weighted duvet 10 is across or approximately perpendicular to the body of the duvet user 1 resting under the weighted duvet 10. Meanwhile it should be understood that a duvet user 1 is likely to move to some extend while using the weighted duvet 10 and thus the placement will not remain completely static during use. Furthermore, different duvet users 1 may have different preferences regarding positions in which the rest and thus how exactly the weighted duvet 10 is placed over them.

Both the first 60 and second lightweight zone 62 have a lower average mass than the central weighted zone 68 extending from zone edge to zone edge 65. In an embodiment of the invention, there are no weight elements 100 in the first 60 or second lightweight zones 62. The first 60 and second lightweight zones 62 having no weight elements 100 will still comprise at least a first 20 and second 30 fabric layer which the weighted duvet 10 is made from. In addition, a filling material 25 may also be present in the lightweight zones 60,62. These materials will naturally contribute to the mass present in the lightweight zones 60,62, meanwhile they will still lead to a significantly lower mass being applied to the duvet user 1 in regions across the ankles and chest where the weight may cause discomfort or other problems for the user.

In other embodiments of the invention, there can be lightweight elements 101 present within the first 60 and/or second lightweight zone 62, those lightweight elements 101 having a lower mass than the weight elements 100 used in the central weighted zone 68, i.e. placed in the duvet sections 52. In the case where lightweight elements 101 are placed in the first 60 and/or second lightweight zones 62, said lightweight zones 60,62 may also have sectioning stiches 52 for guiding the lightweight elements, similar to the duvet sections 50 in the central weighted zone 68.

In Fig. 2, the first 60 and second lightweight zones 62 are shown to be of the same length, but it should be understood that this needs not be the case and the first lightweight zone 60 may be longer or shorter than the second lightweight zone 62 in different embodiments of the invention. In one embodiment of the invention, both the first 60 and the second lightweight zone 62 extend at least 10 cm from their neighbouring edge, i.e. the first lightweight zone 60 extends at least 10 cm from the first edge 11 and the second lightweight zone 62 extends at least 10 cm from the second edge 12. In another embodiment of the invention, the both of the lightweight zones 60,62 extend at least 20 cm from their neighbouring edges. In another embodiment of the invention, the both of the lightweight zones 60,62 extend at least 30 cm from their neighbouring edges.

The weighted duvet illustrated in Fig. 2 further comprises a dividing means 40 allowing the weighted duvet 10 to be divided into two halves. The dividing means 40 may be anything that can releasably hold together the two halves of the weighted duvet 10 such as a zipper, buttons or a string drawn through rivets. Being able to divide the weighted duvet 10 into multiple smaller pieces has several benefits. Due to the nature of the weighted duvet, it is relatively heavy and various embodiments have weights in the range of 3-15 kg. This makes cleaning of the weighted duvet 10 difficult as many washing machines and driers are not able to handle such heavy loads, thus being able to divide the weighted duvet 10 into smaller pieces has the benefit of making the process of cleaning the weighted duvet 10 easier as it can be done in two steps with a smaller total load each. Furthermore, being able to divide the weighted duvet 10 into two separate sections also makes it suitable for more versatile use, for example the connected weighted duvet may be suitable for the duvet user 1 to sleep under, while using only one part of the divided weighted duvet may be more suitable for draping around the shoulders of a duvet user 1 sitting upright or draping across the lap of the duvet user 1 while avoiding that big parts of the weighted duvet 10 has to lie on the floor next to the duvet user 1.

While the invention is illustrated with a single dividing means 40 located at the middle of the weighted duvet 10, it is to be understood that the weighted duvet 10 may have multiple dividing means allowing it to be divided into more than two pieces.

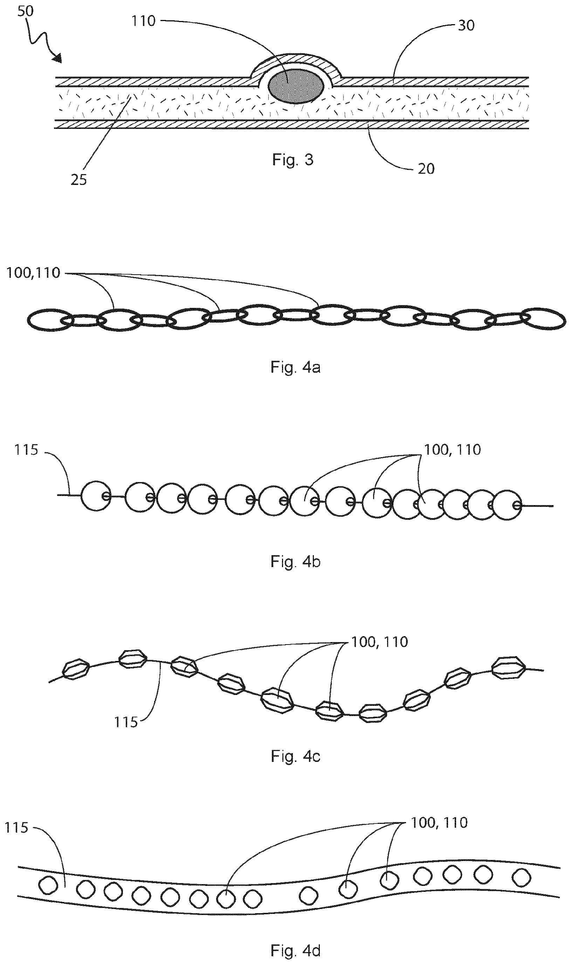

Fig. 3 illustrates the various layers of a preferred embodiment of the weighted duvet 10 shown in a semi-exploded cross-sectional view of a duvet section 50.

The weighted duvet 10 comprises at least a first fabric layer 20 and a second fabric layer 30 (both shown hatched in Fig. 3). In other embodiments, the weighted duvet 10 may comprise additional fabric layers to add warmth, softness or similar beneficial effects. Additional fabric layers may also be functional layers added to contribute specific features to the surface of the weighted duvet 10 on either or both sides, such a layer may for example be particularly insulating making the weighted duvet 10 warmer, it may be particularly soft providing the weighted blanket 10 with a comfortable feel for the duvet user 1, or it may be made from a material with particularly good heat conductivity providing a cooling effect for the duvet user 1. The material of the first 20 and second fabric layer 30 may be the same or it may differ. In a preferred embodiment, the first 20 and second fabric layers 30 are made from the same material being geon.

Between the first 20 and second fabric layer 30, the weighted duvet 10 may comprise a filling material 25 such as fibre filling, down or pieces of foam (illustrated in Fig. 3 as scattered lines). In a preferred variant, the filling material is polyester. This filling material may provide an insulating effect and helps keeping the duvet user 1 warm under the weighted duvet 10. In other embodiments, the filling material 25 may be chosen to be mainly soft and impact absorbent without providing a significant amount of insulation so that the weighted blanket can be used in warmer ambient temperatures. Furthermore, the filling material 25 does also provide friction against the weight elements 100 hampering their movement, while the filling material 25 is still flexible enough that it and the weight elements 100 will still be able to follow the shape of the duvet user 1.

In Fig. 3 the weight element is shown in the form of a connected weight element 110 shown in the cross-sectional view as it is placed along the length of a duvet section 50 in the form of a channel. The connected weight element 110 is placed between the first fabric layer 20 and the second fabric layer 30. Weight elements 100 that are loose would similarly be placed between the first fabric layer 20 and second fabric layer 30, but in that case multiple weight elements 100 would likely be present in the same cross section of the duvet section 50. In Fig. 3, the connected weight elements 110 are illustrated conceptually; Fig. 3 is neither to scale nor showing a specific type of connected weight elements 110.

The connected weight elements 110 may be placed on top of the filling material 25 as shown in Fig. 3, and in this way the duvet user 1 may flip the weighted duvet 10 to obtain different tactile stimuli from the weight elements 100 in the weighted duvet 10. If the layer structure is as shown in Fig. 3, the first fabric layer 20 is neighboured by the filling material 25 which in turn has the weight element 100 placed on it - and possibly sinking slightly into the filling material 25, the structure is then closed off by the second layer 30. Thus, if the second fabric layer 25 is facing the duvet user 1, only limited filling material 25 will be present between the duvet user 1 and the connected weight elements 110. This has the benefit of the duvet user 1 very clearly feeling tactile variations along the connected weight elements 110, thereby getting further stimuli.

In another embodiment of the invention, the discrete segment 110 may be placed amongst the filling material 25 so that it is surrounded by the filling material 25, whereby connected weight elements 110 - or in some embodiments loose weight elements 100 - will experience additional friction and be less likely to move separately and will more thoroughly follow the movements of the entirety of the weighted duvet 10.

The duvet user 1 may also place the weighted duvet 10 such that the filling material 25 is between the duvet user 1 and the connected weight elements 110. This will cause a more even spread of the weight supplied by the connected weight elements 100 leading to a gentler pressure sensation.

The filler material 25 may be any compactable and flexible material, such as fibres, foam pieces or down.

Although not illustrated in Fig. 3, it is foreseen that additional fabric layers may be added on both or either side of the illustrated structure. This can further contribute to having a specific functionality on both sides of the weighted duvet 10. Additional layers may also be used to provide extra padding increasing the even distribution of the pressure feeling through the weighted duvet 10.

No retaining means are included in the illustration of Fig. 3, just as the sectioning stitches defining the edges of the duvet section have not been included in the illustration.

Figs. 4a-4d illustrate various embodiments of connected weight elements 110 according to the invention. These types represent various ways of connecting the weight elements 100 and of delivering different types of tactile and pressure stimuli to the duvet user 1. It is to be understood that they can be combined or varied and are not an exhaustive list. For example, size, mass and material of the connected weight elements 110 and the connection mediators 115 may vary between weight elements 100 located in different duvet section 50 as well as within the same duvet section 50.

Fig. 4a shows a variant of the connected weight elements 110, where the connected weight elements 110 are directly connected to each other as they are linked elements in the form of a chain. Each link is a weight element 100 connected directly to a neighbour link in the chain. In a preferred embodiment, these chains are made of metal, e.g. stainless steel, iron, brass, or zinc. In other equally preferred embodiments, the chains are made from other materials such as glass, wood, or hard plastic which is preferably recycled plastic. The links of the chain may also be made from flexible materials such as pieces of rope or other spun materials of natural fibres, e.g. cellulose fibres. They can also be made from multiple materials by having links either of different materials or by having links made from one material with a coating of another, e.g. a metal core with a rubber cladding.

Fig. 4b shows a variant of the connected weight elements 110 where the weight elements 100 are beads with a hole in them through which they can be placed onto a connection mediator 115. The beads may be spaced apart so that they can move respectively to each other or they may be placed so close to each other that they are in contact and their relative movement is more restricted. Thus, the connected weight elements 110 in the form of beads are not directly linked, however their placement is still related through the connection mediator 115.

The connected weight elements 100 of Fig. 4b, i.e. the beads, may be made from various materials including, but not limited to, plastic, glass, metal, or wood. Although they are illustrated as round with a hole placed centrally, they may take any shape and the hole through which the connection mediator 115 is drawn may also vary in size, shape, and placement. By having the holes placed off-centre they will create an even more varying tactile stimulation.

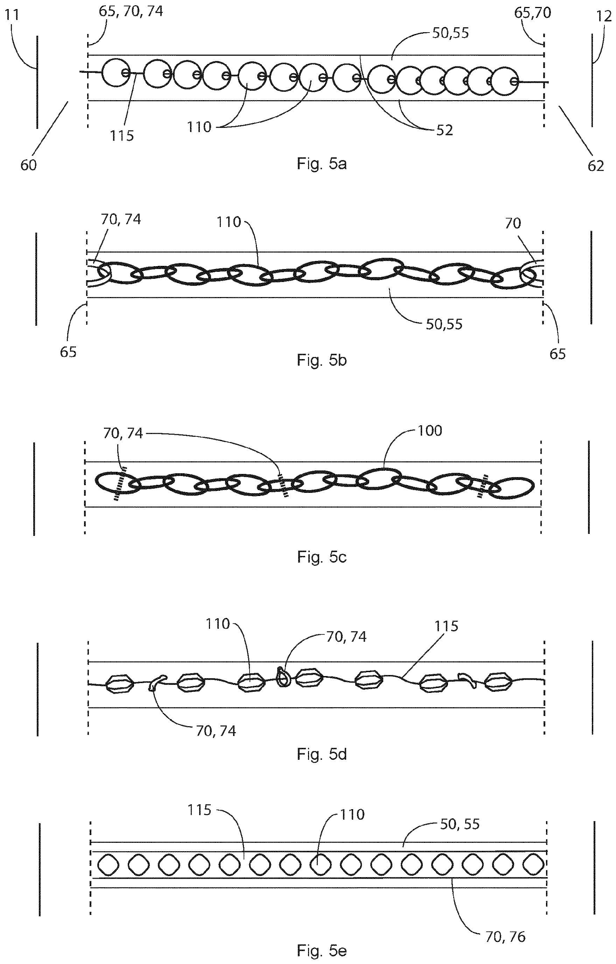

Just as the connected weight elements 110, the connection mediator 115 may be made from different materials like a string of leather, a natural fabric, such as cotton or hemp, or it may be a synthetic string, such as rubber that may give it more elastic properties. The connection mediator 115 could also be metal. Indeed, it would be possible to have a small chain such as the one illustrated in Fig. 5a with the addition of beads placed on it similar to what is shown in Fig. 5b.

Fig. 4c shows a different type of beads on a string being another version of connected weight elements 110 in accordance with the invention. In this version, the weight elements 100 are clamped onto the connection mediator 115, such that the connected weight elements 110 cannot move relative to each other along the connection mediator 115. Such a solution ensures that the pressure delivered by the connected weight elements 110 is pointwise creating more variation across the duvet user 1 and that they will not bunch on the same region of the connection mediator 115.

As illustrated in Fig. 4c, the connected weight elements may have shapes that comprise variations, such as corners, that makes the sensation of pressure even more pointwise. The shown shapes are only illustrative; stars, rectangles, spheres, pyramids and other sorts of shapes are foreseen variations to the invention. Additionally, they may also vary in size along the length of the piece of connected weight elements 110 or they may have the same size.

As previously described, the materials of the connected weight elements 110 and the connection mediator 115 may vary in this embodiment too.

Fig. 4d shows a variant of the connected weight elements 110 that also includes pointwise weights placed statically along a connection mediator 115. In this case, the connection mediator 115 is a band or ribbon of material. Having a band, rather than a string to which the weight elements 100 are attached, has the benefit of a more sturdy set of connected weight elements 110 that is less likely to tear if for example the duvet user 1 pulls on the weighted duvet 10. In this case, the connection mediator may also contribute significantly to the weight of the weighted duvet 10. However, the broader connection mediator 115 may be slightly less flexible depending on the material of which it is made.

Figs. 4a-4d are illustrating only sections of the connected weight elements 110. Although some of them may be mounted directly within the weighted duvet 10 without further treatment, e.g. the chain from Fig. 4a or the clamped beads from Fig. 4c, others may need further treatment. For example, the beads on a string as illustrated in Fig. 4b would include means of stopping the weight elements 100 from falling off the connection mediator 115. Such stopping means may include knots, stitching openings together, or stoppers in the form of static weights at the ends.

Figs. 5a-5e illustrate different embodiments of the retaining means 70 as shown for a single duvet section 50 of the weighted duvet 10 limited by sectioning stitches 52. Note that the illustrations are not to scale and that the duvet sections 50 will commonly be longer than they appear in Figs. 5a-5e in relation the size of the weight elements 100. The same type of retaining means 70 may be used in all duvet sections 70 across the entire weighted duvet 10 or different duvet sections 50 may comprise different retaining means 70. It is also possible that the weighted duvet 10 has duvet sections 50 wherein there are no retainage means 70 and that it has others where there is a combination of multiple retaining means 70 within the same duvet section 50.

Figs. 5a-5e show various forms of retaining means 70, Figs. 5a-5d specifically shows retaining means in the form of point fasteners 74 that keep the connected weight elements 110 in place at specific points within the duvet section 50. Point fasteners 74 may be located at the ends of the connected weight elements 110 or the connection mediator 115, thereby allowing the connected weight elements 110 more freedom between these end points while still being delimited by the sectioning stitches 52 of the duvet section 50. Alternatively, the point fasteners 74 may be placed at multiple positions along the connected weight element 110, thereby restricting its movement further.

Fig. 5a illustrates retaining means 70 in the form of the stitched border of the zone edge 65 to the first 60 and second lightweight zone 62, respectively. These stitched zone edges 65 may be simple lines holding together the first 20 and second fabric layer 30 preventing the connected weight elements 110 from passing a specific position. The stitched zone edge 65 may be a straight line as illustrated or it may have any other geometry potentially leading to varying lengths of different duvet sections 50.

Fig. 5b shows point fasteners 74 in the form of straps. These straps create loops through which connected weight elements 110 are placed. In the illustrated embodiment, the loops are large enough to allow the discrete segment 110 freedom of movement and rotation while connected to the point fastener 74 while still ensuring that some of the weight elements 100 or the connection mediator 115 must be positioned within each loop of the point fasteners 74. The retaining means 70 ensure that the connected weight elements 110 cannot fully bunch in one end of the duvet section 50.

The point fasteners 74 may be straps stitched into the stitches of the zone edge 65 as shown in Fig. 5b, but they may also be straps creating loops stitched to other positions in the duvet section 50 as shown in Fig. 5d. Additionally, the straps of Fig. 5d are shown to be smaller than the weight elements 100 clamped onto the connection mediator 115, such that the connection mediator 115 may move under the point fasteners 74 while the weight elements 100 cannot pass the straps of the point fastener 74. Using smaller straps will restrict the movement of the connected weight elements 110 and give further control of the placement of the weight applied to the duvet user 1. However, since the connection mediator 115 can move with respect to the point fasteners 74 this does not hamper the weighted duvet 10 from conforming to the shape of the body of the duvet user 1.

Fig. 5c shows that point fasteners 74 may be stitches sewing the connected weight elements 110 directly to the second fabric layer 30. Stitches have the benefit of ensuring full control of the placement of weight at specific points along the weighted duvet 10, in this manner it is possible to ensure a spread of the weights or to ensure that weights are retained in spots where they are most needed. However, they do also make specific points of strain to the second fabric layer 30. Meanwhile, knowing these points of stress in advance does also offer the possibility of reinforcing those regions and prevent tears. Such prevention of tears may be done by applying a third fabric layer (not shown) or choosing the second fabric layer in a sturdy material and then applying a different third fabric layer with characteristics chosen not for wear but for the comfort of the duvet user 1.

Fig. 5d illustrates that the retaining means 70 may be a combination of different types of point fasteners 74. In the particular illustration of Fig. 5d, the point fasteners 74 are the stitches of the zone edge 65 as well as multiple types of straps, i.e. traps fastened to the second fabric layer 30 at two point, one on either side of the connection mediator 115, and a loop of a strap fastened to the second fabric layer 30 in a single point. The retaining means 70 are placed in various positions along the duvet section 50. The loops may further comprise clasping means 75 such as snap fasteners or buttons allowing the straps to be opened and closed such that they can provide a tight fit around the connected weight elements 110 that does not allow the weight elements 100 themselves to pass the point fastener 74 and so that the connected weight elements 110 can be taken out or exchanged without breaking the point fastener 74 or the connection mediator 115.

Fig. 5e illustrates an alternative form of retaining means 70 as the discrete segments 110 are connected to the second fabric layer 30 by an adhesive 76 applied between the discrete segments 110 and the second fabric layer 30.

The retaining means 70 of an adhesive 76 may be combined with the point fasteners 74 of stitching the connection mediator 115 into the zone edge 65 allowing a more secure fastening of the connected weight elements 110.

It should be understood that all the types of retaining means 70 can be combined in various ways both along the duvet section and at its ends.

Figs. 6a-6f illustrate various distributions of duvet sections 50 in the central weighted zone 58 as well as placement of weight elements 100 within some weighted sections 55, while other duvet sections 50 remain unweighted. While not shown in Figs. 6a-6f, in some embodiments of the invention all duvet sections 50 will be weighted section 55 containing eight elements 100 of the same type. Similarly, the illustrations of the figures show that it is possible to have varying numbers of duvet sections 50, however, the number of duvet sections 50 illustrated should not be seen as limiting.

In other embodiments of the invention, more or fewer duvet sections may be present depending on the intended size and restriction of the connected weight elements 110.

In Figs. 6a-6f, the weighted elements 100 are not illustrated as such, rather duvet sections 50 with no weight elements are shown as white, while weighted sections 55 are shown as hatched, different hatchings indicate different types and/or average mass of the weighted elements 100 between respective weighted sections 55,55'. Different types of retaining means 70 may be used in the different weighted section 55 regardless of the types of weight elements 100 being the same.

Fig. 6a shows an embodiment of the weighted duvet where the duvet sections 50 are intermittently weighted sections 55 and duvet sections 50 without any weight elements 100. The weighted duvet 10 has dividing means 40 placed centrally on the weighted duvet 10 allowing the weighted duvet 10 to be split into two smaller duvets of equal size. The alternating presence of weight elements 100 in the duvet sections 50 is changed at the centre of the weighted duvet 10 where the weighted sections 55 are present on both sides of the dividing means 40.

Alternating empty duvet sections 50 and weighted sections 55 has the benefit of allowing varying weight stimulation to the duvet user 1 giving a tactile sensation. Furthermore, it allows deep stimuli applied at the weighted sections 55 while the average weight of the full weighted duvet 10 does not become too high to be comfortable for the duvet user 1.

The first 60 and second lightweight zone 62 do not contain any weight elements 100. The first lightweight zone 60 is split on the middle by the separation means 40 into two parts of the first lightweight zone 60,60', however this does not affect the beneficial functionality of having a less heavy region of the weighted duvet 10 at the first edge of the weighted duvet 10. The same is the case for the division of the second lightweight zone 62,62' by the separation means 40.

Fig. 6b shows an embodiment of the weighted duvet 10 in which there are several weighted sections 55 and several duvet sections 50 without any weight elements 100 inside them. Multiple weighted sections 55 are present adjacent to each other at the centre of the weighted duvet 10, while closer to the third 13 and fourth edge 14 of the weighted duvet 10 more duvet sections 50 without any weight elements 10 are present among the weighted sections 55. This distribution of weighted sections 55 leads to the mass of the weighted duvet 10 being largest closest to the middle of the weighted duvet, when the weighted duvet 10 is in full size as the two parts are connected by the dividing means 40. In this manner, the pressure and stimuli provided by the weight elements 100 is greatest at the middle of the weighted duvet 10 allowing the duvet user 1 to get the benefits of the weighted elements 10 while allowing more flexibility of the weighted duvet 10 at the edges, this added flexibility makes it easier for the duvet user 1 to wrap the weighted duvet 10 around them and arrange it for most comfort. Simultaneously, the lower mass near the third 13 and fourth edge 14 of the weighted duvet 10 has similar benefits to those provided by the first 60 and second lightweight zone 62, so that the weighted duvet 10 can be placed such that it does not apply stress to wrist and elbow joints depending on how the duvet user 1 lies under the weighted duvet 10. Furthermore, if the duvet user 1 unintentionally turned the weighted duvet 10 while using it, they would not accidentally place high strain on the regions intended to be under the lightweight zones 60,62.

Fig. 6b further illustrates that it is within the scope of the invention to have distribution of weighted section 55 that is not symmetrical around the dividing means 40. This allows for further customisation to the particular duvet user 1, allowing the weighted duvet 10 to fit to the positions in which the duvet user 1 most frequently lies in. Asymmetric distribution of the weighted sections 55 is also beneficial for weighted duvets 10 shared by multiple users as different duvet users 10 may have different preferences for the optimum amount of weight and stimuli provided by the weighted duvet 10.

While non-symmetrical distribution of the weighted elements 100 is only illustrated in Figs. 6b and 6e, it should not be considered restricted to these particular variations of mass distribution, rather any number of duvet sections and different weight elements 100 may be combined on the two sides of the weighted duvet connected by the dividing means 40.

Fig. 6c shows a weighted duvet 10 with multiple different types of weight elements 100 present in different weighted sections 55,55' present in addition to duvet sections 50 with no weight elements 100. In an embodiment of the invention, the weighted sections 55' placed at the middle of the weighted duvet 10 have weight elements 100' with a higher average mass than the weight elements 100 placed in the weighted sections located closer to the third 13 and fourth edge 14 respectively. Such a distribution of weight has similar benefits as previously described for fewer weighted sections 55 at the third 13 and fourth edge 14 of the weighted duvet 10, however by having different weighted sections 55,55' it is possible to provide more stimuli than in the case of fewer weighted sections 55 while still varying the load applied by the weighted duvet 10 across the width of the weighted duvet 10. Note that in other embodiments of the weighted duvet 10 there may be a variation of the average mass of the weight elements 100,100' distributed across the weighted duvet 10 having different types of weighted sections 55,55' while having no duvet sections 50 without any weighted elements 100 inside. Thus, a higher average mass of the weighted duvet 10 may be obtained while obtaining the benefits of varying mass distribution across the weighted duvet 10.

In other embodiments of the invention, the centrally placed weighted section 55' may have a lower average mass than the weighted section 55 closer to the third 13 and fourth edge 14. This has the benefit of allowing a gentle pressure on the duvet user 1 lying under the middle of the weighted duvet 10 while the duvet user experiences a snug sensation of the weighted duvet 10 around them. Furthermore, the weighted duvet being heavier at the third 13 and fourth edge 14 will provide the weighted duvet 10 with a pressure while the weight elements 100' in the central weighted sections 55' will not provide a high local pressure which can give the duvet user 10 a feeling of a more even distribution of the pressure which some may find more comfortable.

Fig. 6d illustrates a weighted duvet 10 with fewer weighted sections 55 than duvet sections 50 without any weight elements 100 within them. Such a weighted duvet 10 may be particularly useful for sensitive users that benefit from the calming effects of the weighted duvet 10, but would be uncomfortable or experience other adverse effects such as chafing or joint aches under a heavy weighted duvet 10. This type of weighted duvet with few weighted sections 55 may also be beneficial for children whose bodies should not be subjected to as much weight as that of an adult.

Fig. 6d further shows and embodiment of the weighted duvet 10 where only the outermost weighted sections 55' have a different average mass of the weight elements 100' than the weighted sections 55 of the rest of the central weighted zone 58 of the weighted duvet 10. In a preferred embodiment of the invention, the weighted sections 55' adjacent to the third 13 and fourth edge 14, respectively, have a higher average mass of the weight elements 100' than the other weighted sections 55 of the weighted duvet. By having heavier weighted sections 55' at the very edges of the weighted duvet 10, they help keep the weighted duvet 10 in place around the duvet user 1, i.e. the weighted duvet 10 will not easily slide off of the duvet user 1 and the edges will be held down so that they do not fold up and let cold air in under the weighted duvet 10. Keeping the weighted duvet 10 in place around the duvet user 1 may also increase the calming effect as it becomes more cumbersome for the duvet user 1 to remove the weighted duvet 10 and accidentally pull it off if initially moving around before calming down.

Fig. 6d also shows a possible embodiment of the invention wherein there is no dividing means 40 and the weighted duvet 10 is instead a single piece. Having the weighted duvet 10 in a single piece has the benefit of not having a connection means 40 which could break during wear or which provides an unintentional and less controllable tactile stimuli.

Fig. 6e illustrates a weighted duvet with duvet sections 50 that are in the form of smaller pockets rather than being channels spanning the full length between the zone edges 65. The smaller duvet sections 50 may be weighted sections 55 comprising either loose weight elements 100 or connected weight elements 110. In the case of connected weight elements 110, they may benefit from being connected in a loop or having multiple point fasteners rather than being stretched out on a long connection mediator 115 when the duvet sections 50 are not elongated. Similarly, smaller duvet sections 50 as in Fig. 6f may be ideal for loose weight elements 100 as they restrict the movement of weight elements 100 to smaller regions by the sectioning stitches 52. Furthermore, increasing the number of duvet sections 50 in both the lengthwise and transverse direction of the weighted duvet 10 allows for more customisation of the placement of the weighted elements 100. For examples, as illustrated in Fig. 6f, it is possible to have duvet sections 50 without any weight elements centrally next to the zone edges 65 thereby enhancing the effect of lessening the pressure at the sensitive regions of the upper chest and at the ankles. Similarly, other sensitive regions such as at the knees or hips could also have less or no weight elements 100, thereby allowing further customisation of the weighted duvet 10.

Embodiments wherein loose weight elements 100,100' with differing average mass is present in different weighted section 55,55' of smaller duvet sections 50 dividing the central weighted zone 58 in both the longitudinal and transverse directions are also foreseen within the invention.

Fig. 6f shows an embodiment of the weighted duvet 10 for which the weighted elements 100 of the central weighted zone 58 of the weighted duvet 10 are distributed in duvet sections 50 in the form of channels parallel to the first 60 and second lightweight zones 62. In the illustrated embodiment of the invention, the weighted sections 55 contain connected weight elements 110. Having connected weight elements 110 running parallel to the first 60 and second lightweight zones 62, and thus also across the body of the duvet user 1, allows customisation of the pressure along other regions of the body of the duvet user than along the length, e.g. lighter at the knees or hip than across the abdomen and thighs. Having the weight elements 100 distributed across the body of the duvet user 1 also has the benefit of further restricting the movement of the weight elements 100 along the body of the duvet user 1. If the duvet user 1 resting underneath the weighted duvet 10 moves, weight elements are unlikely to shift off of the body of the duvet user 10, instead they will simply shift to a different position on the duvet user 1.

Fig. 6f further illustrates that there may be lightweight elements 101 located in the lightweight zones 60,62 of the weighed duvet 10. In the embodiment illustrated in Fig. 6f, lightweight elements 101 are present only in the first lightweight zone 60. In the particular embodiment as illustrated, the first lightweight zone 60 is divided into a number of weighted sections 55' with lightweight elements 101. The lightweight elements 101 may for example be loose granulate or small beads held in place by the sectioning stiches 52 of the weighted sections 55' of the first lightweight zone 60. Not all duvet sections 50' of the first lightweight zone 60 needs to contain lightweight elements 60. The placement of the lightweight elements 101 may be customised to obtain different benefits, for example by providing a scarce distribution or by adjusting the placement, as in the illustrated case, such that there are no lightweight elements centrally where the weighted duvet 10 is likely to rest at the neck of the duvet user 10 while lightweight elements at near the third 13 and fourth 14 edge of the weighted duvet 10 ensure that a gentle weight is still applied to the shoulders of the duvet user 10. The amount of or density of the lightweight elements 101 are chosen such that the average weight per area in the first 60 and second lightweight zone 62 respectively are lower than the average weight of the central weighted zone 58.

Although not shown in the figures, variants where there are lightweight elements 101 in both the first 60 and second lightweight zone 62 or only in the second lightweight zone 62 are expected embodiments. Furthermore, a lightweight zone 60,62 may be divided by sectioning stitches 52 even if no lightweight elements 101 are present in that lightweight zone 60,62 as shown for the second lightweight zone 62 in Fig. 6f. Although not shown, all weighted sections 55' of the first 60 and/or second lightweight zone 62 may be filled with lightweight elements 101. Lightweight elements 101 may also be connected lightweight elements and generally take any form or be connected by any means described for the weight elements 100 as long as their weight provides the lower average weight in the first 60 and second lightweight zone 62 than in the central weighted zone 58.

Different combinations of the described embodiments are envisaged, thus different types of retaining means 70 may be used in combination, both in different duvet sections 50 and within the same, just as any number of weight elements 100, loose or connected, may be used in the same weighted duvet 10 and the distribution of duvet sections 50 may be varied between different embodiments of the weighted duvet 10 and still being envisioned as a variant of the invention, if they fall within the scope of the claims as the invention is defined by the claims.

## Claims

1. A weighted duvet (10) for applying stimulating pressure to one or more duvet users (1) comprising:
- a first edge (11) positioned opposite a second edge (12) and a third edge (13) positioned opposite a fourth edge (14);
- a first fabric layer (20), a second fabric layer (30);
- at least one duvet section (50) in the central weighted zone (58) of the weighted duvet (10);
- at least one weight element (100) placed between said first (20) and said second fabric layer (30) and within said at least one duvet section (50);
wherein the weighted duvet (10) further comprises a first lightweight zone (60) adjacent to and spanning the length of said first edge (11) and a second light weight zone (62) adjacent to and spanning the length of said second edge (12),
**characterised in that**
the first lightweight zone (60) extends at least 10 cm from the first edge (11) and the second lightweight zone (62) extends at least 10 cm from the second edge (12).

2. A weighted duvet according to claim 1, wherein the first (60) and second lightweight zone (62) are placed at the ends of the weighted duvet (10) with respect to the length (L) of the weighted duvet (10) and said first (60) and second lightweight zone (62) span the width (W) of the lightweight duvet (10).

3. A weighted duvet according to any of the previous claims, wherein neither the first lightweight zone (60) nor the second light weight zone (62) contains any weighted element (100).

4. A weighted duvet according to any of the preceding claims, wherein said at least one duvet section (50) is delimited by sectioning stitches (52) extending through said first fabric layer (20) and said second fabric layer (30) and said first lightweight zone (60) and said second light weight zone (62) are delimited by zone edges (65).

5. The weighted duvet according to any of the preceding claims, wherein weight elements (100) having at least two different average weights are placed in at least two different duvet sections (50).

6. A weighted duvet according to any of the preceding claims, wherein there are at least two duvet sections (50,50') one of said duvet sections (50) comprising at least one weight element (100), thereby being a weighted section (55), and another of said duvet section (50) containing no weighted element (100).

7. A weighted duvet according to any of the preceding claims, wherein at least one of said at least one weight elements (100) is a connected weight element (110).

8. A weighted duvet according to claim 7, wherein at least one of said connected weight elements (110) is a chain.

9. A weighted duvet according to any of the claims 7-8, further comprising retaining means (70) for guiding the weight distribution within said at least one duvet section (50).

10. A weighted duvet according to claim 9, wherein said retaining means (70) comprises at least one point fastener (74).

11. A weighted duvet according to any of the previous claims, wherein filling material (25) is present between said first fabric layer (20) and said second fabric layer (30).

12. A weighted duvet according to any of the preceding claims, wherein said weighted duvet comprises a releasable connection means (40) allowing it to be divided into at least two smaller weighted duvets.

13. A weighted duvet according to claim 12, wherein said releasable connection means (40) is at least one zipper running across the entirety of said weighted duvet parallel to at least one of said duvet sections (50).

## Patentansprüche

1. Gewichtete Bettdecke (10) zum Ausüben von stimulierendem Druck auf einen oder mehrere Bettdeckenbenutzer (1), umfassend:
- eine erste Kante (11), die gegenüber einer zweiten Kante (12) positioniert ist, und eine dritte Kante (13), die gegenüber einer vierten Kante (14) positioniert ist;
- eine erste Stoffschicht (20), eine zweite Stoffschicht (30);
- zumindest einen Bettdeckenabschnitt (50) in der zentralen gewichteten Zone (58) der gewichteten Bettdecke (10);
- zumindest ein Gewichtselement (100), das zwischen der ersten (20) und der zweiten Stoffschicht (30) und innerhalb des zumindest einen Bettdeckenabschnittes (50) platziert ist;
wobei die gewichtete Bettdecke (10) ferner eine erste Leichtgewichtszone (60) benachbart zu der ersten Kante (11) und die Länge davon umspannend und eine zweite Leichtgewichtszone (62) benachbart zu der zweiten Kante (12) und die Länge davon umspannend umfasst,
**dadurch gekennzeichnet, dass**
sich die erste Leichtgewichtszone (60) zumindest 10 cm von der ersten Kante (11) erstreckt und sich die zweite Leichtgewichtszone (62) zumindest 10 cm von der zweiten Kante (12) erstreckt.

2. Gewichtete Bettdecke nach Anspruch 1, wobei die erste (60) und die zweite Leichtgewichtszone (62) an den Enden der gewichteten Bettdecke (10) in Bezug auf die Länge (L) der gewichteten Bettdecke (10) platziert sind und die erste (60) und die zweite Leichtgewichtszone (62) die Breite (W) der Leichtgewichtsbettdecke (10) umspannen.

3. Gewichtete Bettdecke nach einem der vorhergehenden Ansprüche, wobei weder die erste Leichtgewichtszone (60) noch die zweite Leichtgewichtszone (62) ein beliebiges gewichtetes Element (100) enthält.

4. Gewichtete Bettdecke nach einem der vorhergehenden Ansprüche, wobei der zumindest eine Bettdeckenabschnitt (50) durch Teilungsstiche (52) begrenzt ist, die sich durch die erste Stoffschicht (20) und die zweite Stoffschicht (30) erstrecken, und die erste Leichtgewichtszone (60) und die zweite Leichtgewichtszone (62) durch Zonenkanten (65) begrenzt sind.

5. Gewichtete Bettdecke nach einem der vorhergehenden Ansprüche, wobei Gewichtselemente (100), die zumindest zwei verschiedene Durchschnittsgewichte aufweisen, in zumindest zwei verschiedenen Bettdeckenabschnitten (50) platziert sind.

6. Gewichtete Bettdecke nach einem der vorhergehenden Ansprüche, wobei es zumindest zwei Bettdeckenabschnitte (50,50') gibt, wobei einer der Bettdeckenabschnitte (50) zumindest ein Gewichtselement (100) umfasst und dadurch ein gewichteter Abschnitt (55) ist und ein anderer von dem Bettdeckenabschnitt (50) kein gewichtetes Element (100) enthält.

7. Gewichtete Bettdecke nach einem der vorhergehenden Ansprüche, wobei zumindest eines von dem zumindest einen Gewichtselement (100) ein verbundenes Gewichtselement (110) ist.

8. Gewichtete Bettdecke nach Anspruch 7, wobei zumindest eines der verbundenen Gewichtselemente (110) eine Kette ist.

9. Gewichtete Bettdecke nach einem der Ansprüche 7-8, ferner umfassend Haltemittel (70) zum Führen der Gewichtsverteilung innerhalb des zumindest einen Bettdeckenabschnittes (50).

10. Gewichtete Bettdecke nach Anspruch 9, wobei das Haltemittel (70) zumindest eine Punktbefestigung (74) umfasst.

11. Gewichtete Bettdecke nach einem der vorhergehenden Ansprüche, wobei Füllmaterial (25) zwischen der ersten Stoffschicht (20) und der zweiten Stoffschicht (30) vorhanden ist.

12. Gewichtete Bettdecke nach einem der vorhergehenden Ansprüche, wobei die gewichtete Bettdecke ein lösbares Verbindungsmittel (40) umfasst, das ermöglicht, in zumindest zwei kleinere gewichtete Bettdecken geteilt zu werden.

13. Gewichtete Bettdecke nach Anspruch 12, wobei das lösbare Verbindungsmittel (40) zumindest ein Reißverschluss ist, der über die Gesamtheit der gewichteten Bettdecke parallel zu zumindest einem der Bettdeckenabschnitte (50) verläuft.

## Revendications

1. Couette lestée (10) permettant d'appliquer une pression stimulante sur un ou plusieurs utilisateurs de couette (1) comprenant :
- un premier bord (11) positionné à l'opposé d'un deuxième bord (12) et un troisième bord (13) positionné à l'opposé d'un quatrième bord (14) ;
- une première couche de tissu (20), une seconde couche de tissu (30) ;
- au moins une section de couette (50) dans la zone lestée centrale (58) de la couette lestée (10) ;
- au moins un élément de poids (100) placé entre ladite première (20) et ladite seconde couche de tissu (30) et à l'intérieur de ladite au moins une section de couette (50) ;
dans laquelle
la couette lestée (10) comprend en outre une première zone légère (60) adjacente à et s'étendant sur toute la longueur dudit premier bord (11) et une seconde zone légère (62) adjacente à et s'étendant sur toute la longueur dudit deuxième bord (12),
**caractérisée en ce que**
la première zone légère (60) s'étend à au moins 10 cm du premier bord (11) et la seconde zone légère (62) s'étend à au moins 10 cm du deuxième bord (12).

2. Couette lestée selon la revendication 1, dans laquelle la première (60) et la seconde zones légères (62) sont placées aux extrémités de la couette lestée (10) par rapport à la longueur (L) de la couette lestée (10) et lesdites première (60) et seconde zones légères (62) s'étendent sur la largeur (W) de la couette légère (10).

3. Couette lestée selon l'une quelconque des revendications précédentes, dans laquelle ni la première zone légère (60) ni la seconde zone légère (62) ne contiennent d'élément de poids (100).

4. Couette lestée selon l'une quelconque des revendications précédentes, dans laquelle ladite au moins une section de couette (50) est délimitée par des coutures de séparation (52) s'étendant à travers ladite première couche de tissu (20) et ladite seconde couche de tissu (30) et ladite première zone légère (60) et ladite seconde zone légère (62) sont délimitées par des bords de zone (65).

5. Couette lestée selon l'une quelconque des revendications précédentes, dans laquelle des éléments de poids (100) comportant au moins deux poids moyens différents sont placés dans au moins deux sections de couette différentes (50).

6. Couette lestée selon l'une quelconque des revendications précédentes, dans laquelle il y a au moins deux sections de couette (50,50'), l'une desdites sections de couette (50) comprenant au moins un élément de poids (100), étant ainsi une section lestée (55), et une autre desdites sections de couette (50) ne contenant aucun élément de poids (100).

7. Couette lestée selon l'une quelconque des revendications précédentes, dans laquelle au moins un desdits au moins un élément de poids (100) est un élément de poids connecté (110).

8. Couette lestée selon la revendication 7, dans laquelle au moins un desdits éléments de poids connectés (110) est une chaîne.

9. Couette lestée selon l'une quelconque des revendications 7 à 8, comprenant en outre des moyens de retenue (70) pour guider la répartition du poids à l'intérieur de ladite au moins une section de couette (50).

10. Couette lestée selon la revendication 9, dans laquelle lesdits moyens de retenue (70) comprennent au moins une attache à points (74).

11. Couette lestée selon l'une quelconque des revendications précédentes, dans laquelle un matériau de remplissage (25) est présent entre ladite première couche de tissu (20) et ladite seconde couche de tissu (30).

12. Couette lestée selon l'une quelconque des revendications précédentes, dans laquelle ladite couette lestée comprend un moyen de connexion libérable (40) lui permettant d'être divisée en au moins deux couettes lestées plus petites.

13. Couette lestée selon la revendication 12, dans laquelle ledit moyen de connexion libérable (40) est au moins une fermeture éclair s'étendant sur la totalité de ladite couette lestée parallèlement à au moins une desdites sections de couette (50).
